(19) Europäisches Patentamt
European Patent Office
Office européen des brevets

(11) Veröffentlichungsnummer: **0 015 567**
**B1**

(12) # EUROPÄISCHE PATENTSCHRIFT

(45) Veröffentlichungstag der Patentschrift: **15.12.82**

(51) Int. Cl.³: **C 07 C125/06**, C 07 C127/19, C 07 C155/02

(21) Anmeldenummer: **80101160.2**

(22) Anmeldetag: **07.03.80**

(54) **Verfahren zur Herstellung von Phenylcarbamidsäurechloriden.**

(30) Priorität: **08.03.79 DE 2909198**

(43) Veröffentlichungstag der Anmeldung: **17.09.80 (Patentblatt 80/19)**

(45) Bekanntmachung des Hinweises auf die Patenterteilung: **15.12.82 Patentblatt 82/50**

(84) Benannte Vertragsstaaten: **BE CH DE FR GB**

(56) Entgegenhaltungen: **DD C 4 482**

(73) Patentinhaber: **BASF Aktiengesellschaft**
**Carl-Bosch-Strasse 38**
**D-6700 Ludwigshafen (DE)**

(72) Erfinder: **Schirmer, Ulrich, Dr.**
**Berghalde 79**
**D-6900 Heidelberg 1 (DE)**

**EP 0 015 567 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von Phenylcarbamidsäurechloriden

Die Erfindung betrifft ein Verfahren zur Herstellung von Phenylcarbamidsäurechloriden durch Umsetzung von N-monosubstituierten Phenylendiaminen mit überschüssigem Phosgen und anschließende Umsetzung mit Thioalkoholen, Alkoholen, Hydroxylaminen oder Aminen.

Es ist aus Houben-Weyl, Methoden der Organischen Chemie, Band 8, Seiten 115 bis 124, und Annalen der Chemie, Band 562 (1949), Seiten 75 bis 136, bekannt, aromatische Amine mit Phosgen zu Phenylisocyanaten umzusetzen ; durch Anlagerung von Chlorwasserstoff, z.B. aus dem Abgas der Reaktion, entstehen die entsprechenden Phenylcarbamidsäurechloride. Im Falle von Diaminen entstehen ebenfalls analoge Diisocyanate. Diamine, die bei der Umsetzung der beiden Aminogruppen unterschiedlich reagieren, werden nicht beschrieben. Isocyanatocarbamidsäurechloride kann man nach der Lehre einer Veröffentlichung in den Annalen (loc. cit., Seite 84) nur beim Phosgenieren der Chlorhydrate aliphatischer, primär-sekundärer Amine erhalten ; es wird lediglich die Herstellung des N-Butylcarbamidsäurechloridhexylisocyanats beschrieben.

Es ist aus der Angewandten Chemie, Band 74 (1962), Seiten 795 bis 799 bekannt, daß man aus Toluylen-2,4-diisocyanat bei Einwirkung äquimolarer Mengen Salzsäure das p-Methyl-m-isocyanatocarbamidsäurechlorid erhält.

Es ist aus der deutschen Patentschrift 873 084 bekannt, durch Phosgenieren von diaminen mit einer primären und einer sekundären Aminogruppe bei erhöhter Temperatur Isocyanatocarbamidsäurechloride zu erhalten. Es wird lediglich die Herstellung des N-Butyl-N-(6-isocyanatohexyl)-carbamidsäurechlorids und des entsprechenden N-(α-Äthylhexyl)-N-(6-isocyanatohexyl)-carbamidsäurechlorids beschrieben. In der gleichen Patentschrift wird darauf hingewiesen, daß aus derartigen Carbamidsäurechloridisocyanaten schon beim Lösen in Äthylalkohol Diurethane gebildet werden.

Die DD-PS 4482 zeigt die Herstellung von mehrfach reaktionsfähigen Carbamidsäurederivaten durch Umsetzung von Aminen betimmter Struktur mit mindestens einem primären und mindestens einem sekundären oder tertiären Stickstoffatom mit Phosgen. Sie beschreibt, daß Diphenylamincarbaminsäurechlorid-4,4'-diisocyanat mit Methanol Diphenylamincarbaminsäurechlorid-4,4'-dimethylurethan bildet. Es bleibt somit, ganz im Gegensatz zu der Lehre der DE-PS 873 084, die Carbamidsäuregruppe erhalten. Im ersten Fall (DD-PS 4482) geht es um die Reaktionen bei Molekülen, die gleichzeitig die Strukturelemente $(Aryl)_2NCOCl$ und Aryl-NCO enthalten, während im zweiten Fall die Strukturelemente $(Alkyl)_2NCOCl$ und Alkyl-NCO nebeneinander vorliegen.

Es wurde nun gefunden, daß man Phenylcarbamidsäurechloride der Formel I

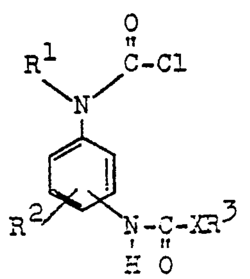

(I)

worin X ein Sauerstoffatom, ein Schwefelatom oder den Rest $-\overset{R^4}{\overset{|}{N}}-$ bedeutet, $R^1$ einen aliphatischen Rest bezeichnet, $R^2$, $R^3$ und $R^4$ gleich oder verschieden sein können und jeweils für einen aliphatischen, cycloaliphatischen, araliphatischen oder aromatischen Rest stehen, $R^4$ auch für ein Wasserstoffatom oder eine Alkoxygruppe stehen kann, $R^2$ auch ein Wasserstoffatom, ein Halogenatom, eine Nitrogruppe oder eine Alkoxygruppe bezeichnen kann, vorteilhaft erhält, wenn man N-monosubstituierte Phenylendiamine der Formel II

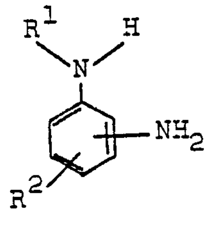

(II)

worin $R^1$ und $R^2$ die vorgenannte Bedeutung besitzen, in einem ersten Schritt mit einem Überschuß von Phosgen bei einer Temperatur von − 30 °C bis + 200 °C umsetzt und ohne Abtrennung in einem zweiten

Schritt die so erhaltenen Isocyanatophenylcarbamidsäurechloride der Formel III

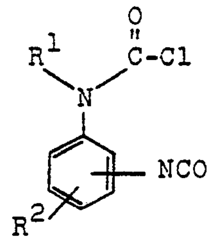

(III)

worin $R^1$ und $R^2$ die vorgenannte Bedeutung besitzen, mit Verbindungen der Formel IV

$$H—X—R^3 \qquad (IV),$$

worin $R^3$ und X die vorgenannte Bedeutung besitzen, umsetzt.

Weiterhin wurden die neuen Phenylcarbamidsäurechloride der Formel I

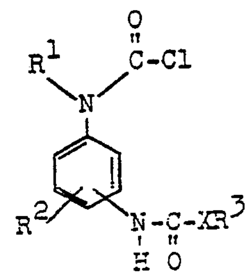

(I)

worin X ein Sauerstoffatom, ein Schwefelatom oder den Rest $-\overset{R^4}{\overset{|}{N}}-$ bedeutet, $R^1$ einen aliphatischen Rest bezeichnet, $R^2$, $R^3$ und $R^4$ gleich oder verschieden sein können und jeweils für einen aliphastichen, cycloaliphatischen, araliphatischen oder aromatischen Rest stehen, $R^4$ auch für ein Wasserstoffatom oder eine Alkoxygruppe stehen kann, $R^2$ auch ein Wasserstoffatom, ein Halogenatom, eine Nitrogruppe oder eine Alkoxygruppe bezeichnen kann, gefunden.

Die Umsetzung läßt sich unter Verwendung von N-Methyl-m-phenylen-diamin und Methanol durch die folgenden Formeln wiedergeben

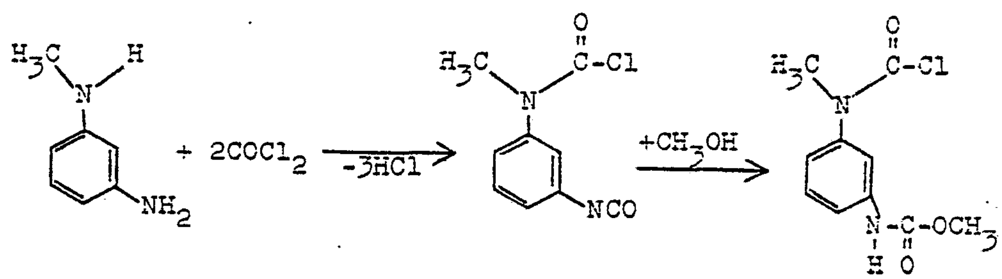


Die Erfindung geht von der Beobachtung aus, daß eine einbadige Umsetzung des Reaktionsgemisches, das bei der Herstellung von Isocyanatophenylcarbamidsäurechloriden III entsteht, mit Verbindungen der Formel IV überraschend vorteilhaft Phenylcarbamidsäurechloride I ergibt. Im Hinblick auf den Stand der Technik liefert das Verfahren nach der Erfindung auf einfachem und wirtschaftlichem Wege eine große Zahl der neuen Phenylcarbamidsäurechloride in guter Ausbeute und Reinheit. Alle diese vorteilhaften Ergebnisse des erfindungsgemäßen Verfahrens sind überraschend, denn man hätte unter Berücksichtigung der vorgenannten Veröffentlichungen gerade bei aromatischen Diaminen eine Umsetzung beider Aminogruppen zu Diurethanen und infolge der einbadigen Arbeitsweise ohne Zwischenisolierung der Stoffe III die Bildung heterogener Gemische zahlreicher Nebenstoffe erwarten müssen. Im Hinblick auf DD-PS 4482 und DE-PS 873 084 waren ebenfalls heterogene Gemische zu erwarten, die einheitliche, erfindungsgemäße Reaktion war daher überraschend.

Die Ausgangsstoffe II lassen sich leicht nach bekannten Verfahren, z.B. nach dem in den Berichten, Band 19 (1886), Seiten 546 bis 551, beschriebenen Verfahren, herstellen. Phosgen wird im Überschuß, vorzugsweise in einer Menge von 2,1 bis 8, insbesondere 2,1 bis 4 Mol Phosgen je Mol Ausgangsstoff II

umgesetzt. Bevorzugte Ausgangsstoffe II und dementsprechend bevorzugte Stoffe III, IV und bevorzugte Endstoffe I sind solche, in deren Formeln X ein Sauerstoffatom, ein Schwefelatom oder den Rest

$R^4$
|
—N— bedeutet, $R^1$ einen Alkylrest mit 1 bis 7 Kohlenstoffatomen oder einen Alkenylrest oder einen Alkinylrest mit jeweils 2 bis 7 Kohlenstoffatomen bezeichnet, $R^2$, $R^3$ und $R^4$ gleich oder verschieden sein können und jeweils für einen Alkylrest oder Chloralkylrest mit jeweils 1 bis 7 Kohlenstoffatomen, einen Cyanakylrest, Alkenylrest oder Alkinylrest mit jeweils 2 bis 7 Kohlenstoffatomen, einen monocyclischen oder bicyclischen Cycloalkylrest mit 5 bis 10 Kohlenstoffatomen, einen Aralkylrest oder Alkylarylrest mit jeweils 7 bis 12 Kohlenstoffatomen, einen, gegebenenfalls durch Halogenatome, insbesondere Fluor- oder Chloratome, substituierten Phenylrest stehen, $R^4$ auch für ein Wasserstoffatom oder eine Alkoxygruppe mit 1 bis 4 Kohlenstoffatomen stehen kann, $R^2$ auch ein Wasserstoffatom, ein Bromatom oder insbesondere ein Chloratom, eine Nitrogruppe oder eine Alkoxygruppe mit 1 bis 7 Kohlenstoffatomen bezeichnen kann. Die beiden Stickstoffatome der Ausgangsstoffe II stehen in o-, zweckmäßig in p- und bevorzugt in m-stellung zueinander. Die vorgenannten Reste können noch durch unter den Reaktionsbedingungen inerte Gruppen oder Atome, z.B. Alkylgruppen oder Alkoxygruppen mit jeweils 1 bis 4 Kohlenstoffatomen ; aromatische Kerne substituierende Fluoratome oder Chloratome ; substituiert sein.

Beispielsweise sind folgende Ausgangsstoffe II geeignet : N-Methyl, N-Äthyl-, N-Propyl-, N-Isopropyl-, N-Butyl-, N-Isobutyl-, N-sek.-Butyl-, N-tert.-Butyl-, N-Allyl-, N-Propargyl-1,2-phenylendiamin, entsprechende 1,4-Phenylendiamine und bevorzugt 1,3-Phenylendiamine ; homologe, in 2-, 3-, 4-, 5- oder insbesondere 6-Stellung durch ein Bromatom, Chloratom, die Nitrogruppe, Methoxy-, Äthoxy-, Propoxy-, Isopropoxy-, Butoxy-, Isobutoxy-, sek.-Butoxy-, tert.-Butoxy-, Methyl-, Äthyl-, Propyl-, Isopropyl-, Butyl-, Isobutyl-, sek.-Butyl-, tert.-Butyl-, Allyl-, Crotyl-, Propargyl-, Cyclopentyl-, Cyclohexyl-, Benzyl-, Tolyl-, Phenyl-gruppe substituierte Phenylendiamine.

Die Umsetzung wird im ersten Schritt bei einer Temperatur von − 30 °C bis + 200 °C, vorzugsweise von − 15 bis + 140 °C, drucklos oder unter Druck, kontinuierlich oder diskontinuierlich durchgeführt. Zweckmäßig verwendet man unter den Reaktionsbedingungen inerte Lösungsmittel. Als Lösungsmittel kommen z.B. in Frage : aromatische Kohlenwasserstoffe, z.B. Toluol, Äthylbenzol, o-, m-, p-Xylol, Isopropylbenzol, Methylnaphthalin ; Halogenkohlenwasserstoffe, insbesondere Chlorkohlenwasserstoffe, z.B. Tetrachloräthylen, 1,1,2,2,-oder 1,1,1,2-Tetrachloräthan, Chloroform, Chlornaphthalin, Dichlornaphthalin, Tetrachlorkohlenstoff, 1,1,1- oder 1,1,2-Trichloräthan, Trichloräthylen, Pentachloräthan, 1,2-Dichloräthan, 1,1-Dichloräthan, Chlorbenzol, Fluorbenzol, Brombenzol, Jodbenzol, o-, p- und m-Dichlorbenzol, o-, m-, p-Chlortoluol, 1,2,4-Trichlorbenzol, 1,10-Dibromdekan, 1,4-Dibrombutan ; Äther, z.B. Äthylpropyläther, Methyl-tert.-butyläther, n-Butyläthyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan, Thioanisol ; Ketone wie Methyläthylketon, Aceton, Diisopropylketon, Diäthylketon, Methylisobutylketon, Mesityloxid, Acetophenon, Cyclohexanon, Äthylisoamylketon, Diisobutylketon, Methylcyclohexanon, Dimethylcyclohexanon ; Nitrokohlenwasserstoffe wie Nitromethan, Nitroäthan, Nitrobenzol, o-, m-, p-Chlornitrobenzol, o-Nitrotoluol ; Nitrile wie Acetonitril, Butyronitril, Isobutyronitril, Benzonitril, m-Chlorbenzonitril ; aliphatische oder cycloaliphatische Kohlenswasserstoffe, z.B. Heptan, α-Pinen, Pinan, Nona, Benzinfraktionen innerhalb eines Siedepunktintervalls von 70 bis 190 °C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan ; und entsprechende Gemische. Zweckmäßig verwendet mas das Lösungsmittel in einer Menge von 400 bis 10 000 Gewichtsprozent, vorzugsweise von 700 bis 1 800 Gewichtsprozent, bezogen auf Ausgangsstoff II.

Die Reaktion des ersten Schrittes kann wie folgt durchgeführt werden : Ein Gemisch von Ausgangsstoff II, Phosgen und gegebenenfalls Lösungsmittel wird während 1 bis 24 Stunden bei der Reaktionstemperatur gehalten. In einer bevorzugten Ausführungsform legt man einen Teil des Phosgens, zweckmäßig 25 bis 75 Gewichtsprozent der Gesamtmenge Phosgen, und das Lösungsmittel vor und gibt den Ausgangsstoff II langsam, z.B. während 0,5 bis 4 Stunden, bei zweckmäßig − 20 bis + 10 °C zu ; dann wird unter Erhitzen auf zweckmäßig 80 bis 150 °C das restliche Phosgen, zweckmäßig während 1 bis 10 Stunden, eingeleitet. Nun wird das Reaktionsgemisch, zweckmäßig nach Entfernung des überschüssigen Phosgens mittels Stickstoff, dem zweiten Schritt der Umsetzung unterworfen. Gegebenenfalls kann ein Teil des Lösungsmittels, vorteilhaft 10 bis 80 Gewichtsprozent der Gesamtmenge an Lösungsmittel, z.B. durch fraktionierte Destillation, ebenfalls vor Beginn des zweiten Schrittes aus dem Reaktionsgemisch abgetrennt werden.

Das so erhaltene Reaktionsgemisch des ersten Schrittes wird im zweiten Schritt mit Alkoholen, Thioalkoholen, N-und O-substituierten Hydroxylaminen oder Aminen der Formel IV, in stöchiometrischer Menge oder im Überschuß, vorzugsweise in einer Menge von 1 bis 2 Mol Stoff IV je Mol Ausgangsstoff II, umgesetzt.

Beispielsweise sind folgende Ausgangsstoffe IV geeignet :

Methyl-, Äthyl-, Propyl-, Isopropyl-, Butyl-, Isobutyl-, sek.-Butyl-, tert.-Butyl-, Allyl-, Crotyl-, Propargyl-, Cyclopentyl-, Cyclohexyl-, Benzyl-, 1,1-Dimethylpropargyl-, Cyanomethyl-alkohol, Phenol, o-Methylphenol, m-Methylphenol, p-Methylphenol ; analoge Thioalkohole ; durch vorgenannte Substituenten gleich

oder unterschiedlich, einfach oder zweifach an Stickstoffatom substituierte Amine sowie entsprechende N-O-disubstituierte Hydroxylamine. Die Umsetzung wird im allgemeinen bei einer Temperatur von – 10 °C bis + 100 °C, vorzugsweise von – 5 bis + 30 °C, drucklos oder unter Druck, kontinuierlich oder diskontinuierlich durchgeführt. Zweckmäßig verwendet man unter den Reaktionsbedingungen inerte Lösungsmittel. Als Lösungsmittel kommen im allgemeinen die für den ersten Schritt der Umsetzung genannten in Betracht.

Der zweite Schritt der Umsetzung kann wie folgt durchgeführt werden : Das Gemisch des ersten Reaktionsschrittes, das den Stoff III enthält, zusammen mit Ausgangsstoff IV und gegebenenfalls zusätzlichen Lösungsmittel wird während 0,5 bis 168 Stunden bei der Reaktionstemperatur gehalten. Dann wird aus dem Reaktionsgemisch der Endstoff in üblicher Weise, z.B. durch Filtration, abgetrennt.

Die nach dem Verfahren der Erfindung erhältlichen neuen Phenylcarbamidsäurechloride der Formel I sind wertvolle Zwischenprodukte für die Herstellung von Pharmazeutika, Farbstoffen, Pflanzenschutzmitteln, Kunststoffen Kunststoff- und Textilhilfsmitteln. So können aus ihnen, z.B. durch Umsetzung mit Alkoholen, Thiolen oder Aminen, die entsprechenden Dicarbamidsäureester, -thioester und Diharnstoffe hergestellt werden, die wertvolle Herbicide darstellen. Es wird auf die US-Patentschrift 3 867 426 und 4 013 450 sowie die deutsche Offenlegungsschrift 27 03 838 und 27 25 146 verwiesen.

Die in den folgenden Beispielen aufgeführten Teile bedeuten Gewichtsteile. Die Gewichtsteile verhalten sich zu den Volumenteilen wie Kilogramm zu Liter.

## Beispiel 1

Bei – 10 bis – 15 °C werden in 200 Teilen Toluol 80 Teile Phosgen vorgelegt. Unter gutem Rühren werden bei – 10 °C 40 Teile N-Methyl-m-phenylendiamin in 300 Teilen Toluol während 1 1/2 Stunden zugegeben. Man erwärnt innerhalb 2 Stunden auf 20 °C, dann innerhalb einer Stunde auf 60 °C und schließlich innerhalb 2 Stunden auf 110 °C, wobei sich unter starker Gasentwicklung eine klare Lösung bildet. Das Gemisch wird unter Rückfluß bei 110 °C noch 4 Stunden unter Einleiten von Phosgen (30 Teile pro Stunde) gehalten. Man engt das Gemisch im Vakuum auf 200 Volumenteile ein, gibt bei 20 °C 18 Teile Äthylalkohol zu, engt das Gemisch nach 24 Stunden Reaktionszeit (Reaktionstemperatur 20 °C) im Vakuum ein, wobei sich ein viskoses Öl bildet, das durch Zusatz von Toluol/Petroläther zum Kristallisieren gebracht wird. Nach Absaugen des Gemischs, Waschen und Trocknen des Filtergutes im Vakuum erhält man 80 Teile (95 % der Theorie) O-Äthyl-N-(3-(N'-methyl-N'-chlorcarbonyl)-aminophenyl)-carbamat vom Fp 63 bis 65 °C.

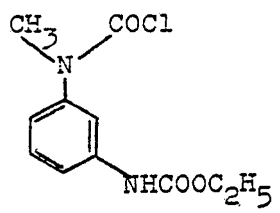

## Beispiel 2

Zu einer auf 200 Volumenteile eingeengten, analog Beispiel 1 hergestellten Lösung von N-(3-Isocyanatophenyl)-N-methyl-carbamidsäurechlorid aus 40 Teilen N-Methyl-m-phenylendiamin gibt man bei 0 °C 14,8 Teile Dimethylamin in 150 Teilen Toluol. Nach zweistündigem Rühren bei 10 °C wird das Gemisch abgesaugt, mit Toluol gewaschen und im Vakuum getrocknet. Man erhält 69 Teile (82 % der Theorie) N,N-Dimethyl-N'-(3-(N''-methyl-N''-chlorcarbonyl)-aminophenyl)-harnstoff vom Fp 76 bis 77 °C.

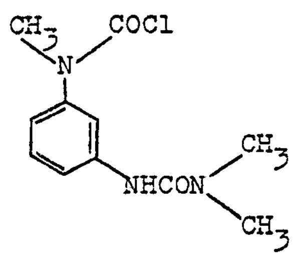

## Beispiel 3 bis 20

Entsprechend diesen Beispielen werden die folgenden Endstoffe I hergestellt (Tabelle), wobei lediglich die Ausgangsstoffe II und IV variiert werden :

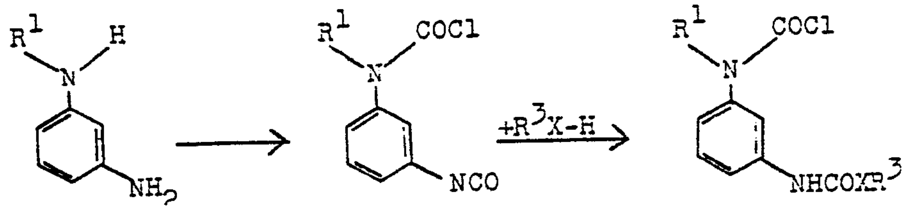


(II) (III) (I)

| Bei-spiel | Ausgangsstoff II $R^1$ = | Teile | Ausgangsstoff IV $R^3X$ = | Teile | analog zu Beispiel | Ausbeute an Endstoff I in % der Theorie | Fp °C von Endstoff I |
|---|---|---|---|---|---|---|---|
| 3 | $CH_3$ | 40 | $CH_3NH$- | 10,2 | 2 | 90 | 157 - 159 |
| 4 | $CH_3$ | 40 | $CH_3S$- | 16,2 | 1 | 84 | 176 - 177 |
| 5 | $CH_3$ | 40 | $(CH_3)_3C$-O- | 29,8 | 1 | 42 | 119 - 121 |
| 6 | $CH_3$ | 40 | $C_2H_5$-S- | 29,4 | 1 | 87 | 97 - 99 |
| 7 | $CH_3$ | 40 | $(CH_3)_2CHO$- | 29,6 | 1 | 100 | hochviskoses Öl |
| 8 | $C_2H_5$ | 44,5 | $CH_3O$- | 10,8 | 1 | 92 | 86 - 88 |
| 9 | $C_2H_5$ | 44,5 | $CH_3S$- | 16,2 | 1 | 84 | 104 - 106 |
| 10 | $CH_3$ | 40 | $(CH_3)_3C$-NH- | 22,6 | 2 | 90 | 178 - 180 |
| 11 | $CH_3$ | 40 | $(CH_3)(CH_3O)N$- | 20 | 2 | 54 | 91 - 93 |
| 12 | $CH_3$ | 40 | 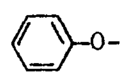 | 30,8 | 1 | 100 | hochviskoses Öl |
| 13 | $CH_3$ | 40 | 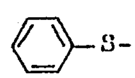 | 54,1 | 1 | 89 | 125 - 126 |
| 14 | $CH_3$ | 40 | 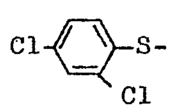 | 71,2 | 1 | 64 | 107 - 108 |
| 15 | $CH_3$ | 40 | 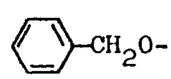 | 40,8 | 1 | 64 | 89 - 91 |
| 16 | $CH_3$ | 40 | 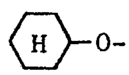 | 42,1 | 1 | 33 | 96 - 97 |
| 17 | $CH_3$ | 40 | 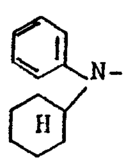 | 65,3 | 2 | 68 | 183 - 184 |
| 18 | $CH_3$ | 40 | 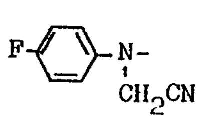 | 49,2 | 2 | 56 | 180 - 182 |
| 19 | $CH_3$ | 40 | 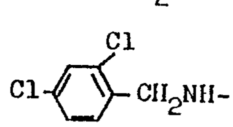 | 57,7 | 2 | 90 | 159 - 160 |
| 20 | $CH_3$ | 40 | $CH_3O$ | 14,4 | 1 | 87 | 118 - 120 |

Beispiel 21 (Verwendung)

Zu 10 Teilen S-Methyl-N-(3'-[N'-methyl-N'-chlorcarbonyl]-aminophenyl)-thiolcarbamat aus Beispiel 4 in 200 Teilen Acetonitril gibt man 5,5 Teile Natrium-4-chlorphenolat in 100 Teile Acetonitril bei 20 °C. Die Temperatur steigt auf 27 °C an. Nach zweistündigem Rühren wird das Gemisch filtriert, eingeengt und aus Toluol umkristallisiert. Man erhält 5,3 Teile einer weißen kristallinen Verbindung vom Fp 149 bis 152 °C mit folgender Strukturformel

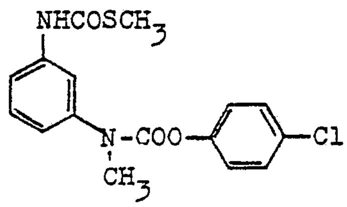

Beispiel 22 (Verwendung)

Zu 13,7 Teilen O-Methyl-N-(3'-[N'-methyl-N'-chlorcarbonyl]-aminophenyl)-carbamat aus Beispiel 20, in 250 Teilen Toluol suspendiert, gibt man zwischen 20 bis 30 °C 5,75 Teile Dimethylamin in 60 Teile Toluol, läßt das Gemisch 2 Stunden bei 30 °C nachrühren, gibt 100 Teile Wasser zu und saugt das Gemisch ab. Nach dem Trocknen erhält man 13,1 Teile einer weißen, kristallinen Verbindung vom Fp 166 bis 167 °C mit folgender Strukturformel

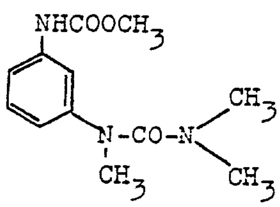

Analog Beispiel 21 bzw. 22 werden folgende Verbindungen hergestellt :

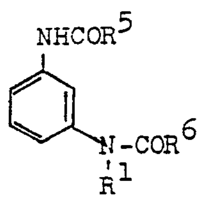

| $R^5$ | $R^1$ | $R^6$ | Fp in °C |
|---|---|---|---|
| $-OCH_3$ | $CH_3$ | 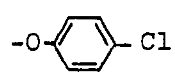 | 183 - 185 |
| 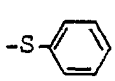 | $CH_3$ | $-OCH_3$ | 163 - 165 |
| 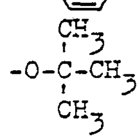 | $CH_3$ | 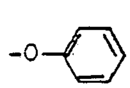 | 134 - 136 |
| 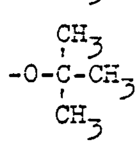 | $CH_3$ | 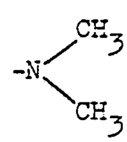 | 145 - 147 |
| $-OCH_3$ | $CH_3$ | 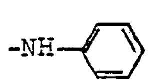 | 170 - 172 |

(Fortsetzung)

| $R^5$ | $R^1$ | $R^6$ | Fp in °C |
|---|---|---|---|
| $-OCH_3$ | $CH_3$ | 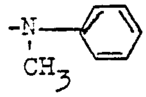 | 172 - 174 |
| $-SCH_3$ | $CH_3$ | 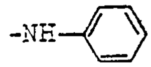 | 167 - 169 |
| $-SCH_3$ | $CH_3$ | 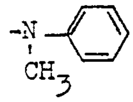 | 123 - 125 |
| 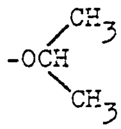 | $CH_3$ | 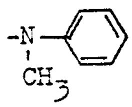 | 157 - 159 |
| $-OC_2H_5$ | $CH_3$ | 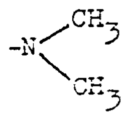 | 116 - 118 |
| $-OCH_3$ | $CH_3$ | 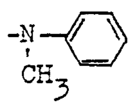 | 94 - 96 |
| $-OCH_3$ | $CH_2CH_2Cl$ | 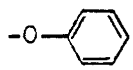 | 141 - 143 |
| $-OCH_3$ | $CH_3$ | 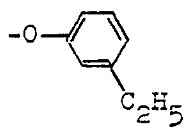 | 98 - 100 |
| $-OCH_3$ | $CH_3$ | 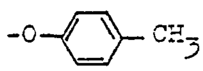 | 130 - 132 |
| $-SCH_3$ | $CH_3$ | 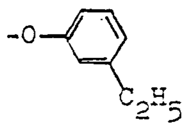 | 123 - 126 |
| 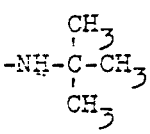 | $CH_3$ | 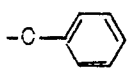 | 186 - 188 |
| $-OCH_3$ | $C_2H_5$ | 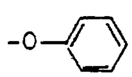 | 129 - 131 |

**Anspruch**

Verfahren zur Herstellung von Phenylcarbamidsäurechloriden der Formel I

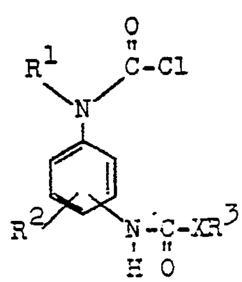

(I)

worin X ein Sauerstoffatom, ein Schwefelatom oder den Rest $-\overset{R^4}{\overset{|}{N}}-$ bedeutet, $R^1$ einen aliphatischen Rest bezeichnet, $R^2$, $R^3$ und $R^4$ gleich oder verschieden sein können und jeweils für einen aliphatischen, cycloaliphatischen, araliphatischen oder einen aromatischen Rest stehen, $R^4$ auch für ein Wasserstoffatom oder eine Alkoxygruppe stehen kann, $R^2$ auch ein Wasserstoffatom, ein Halogenatom, eine Nitrogruppe oder eine Alkoxygruppe bezeichnen kann, dadurch gekennzeichnet, daß man N-monosubstituierte Phenylendiamine der Formel II

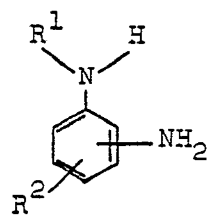

(II)

worin $R^1$ und $R^2$ die vorgenannte Bedeutung besitzen, in einem ersten Schritt mit einem Überschuß von Phosgen bei einer Temperatur von − 30 °C bis + 200 °C umsetzt und ohne Abtrennung in einem zweiten Schritt die so erhaltenen Isocyanatophenylcarbamidsäurechloride der Formel III

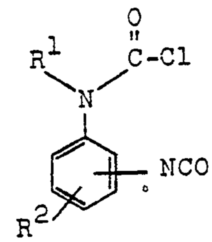

(III)

worin $R^1$ und $R^2$ die vorgenannte Bedeutung besitzen, mit Verbindungen der Formel IV

$$H-X-R^3 \qquad (IV),$$

worin $R^3$ und X die vorgenannte Bedeutung besitzen, umsetzt.

**Claim**

A process for the preparation of a phenylcarbamic acid chloride of the formula I

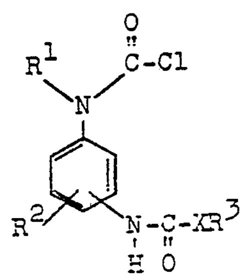

(I)

where X is oxygen, sulfur or $-\overset{R^4}{\overset{|}{N}}-$, $R^1$ is an aliphatic radical, $R^2$, $R^3$ and $R^4$ are identical or different and are each an aliphatic, cycloaliphatic, araliphatic or aromatic radical, $R^4$ can also be hydrogen or alkoxy and $R^2$ can also be hydrogen, halogen, nitro or alkoxy, wherein an N-monosubstituted phenylenediamine of the formula II

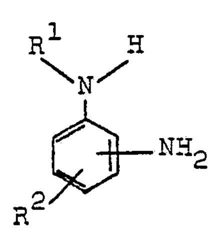

(II)

where $R^1$ and $R^2$ have the above meanings, is reacted, in a first step, with an excess of phosgene at from − 30 °C to + 200 °C and the resulting isocyanatophenylcarbamic acid chloride of the formula III

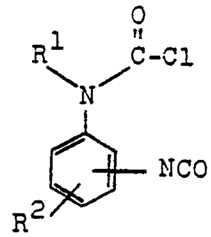

(III)

where $R^1$ and $R^2$ have the above meanings, is reacted in a second step, without prior isolation, with a compound of the formula IV

$$H-X-R^3 \qquad (IV)$$

where $R^3$ and X have the above meanings.

## Revendication

Procédé de préparation de chlorures de phényl-carbamoyle de la formule I

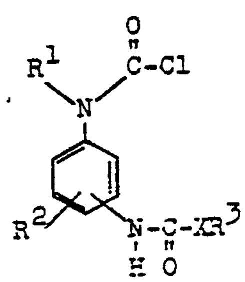

(I)

dans laquelle X représente un atome d'oxygène ou un atome de soufre ou un groupe $-\overset{R^4}{\overset{|}{N}}-$, $R^1$ désigne un reste aliphatique et $R^2$, $R^3$ et $R^4$, qui peuvent être identiques ou différents, désignent chacun un reste aliphatique, cycloaliphatique, araliphatique ou aromatique, $R^2$ pouvant de plus désigner un atome d'hydrogène ou d'halogène ou un groupe nitro ou alcoxy et $R^4$ un atome d'hydrogène ou un radical alcoxy, caractérisé en ce que l'on fait réagir des phénylène-diamines N-mono-substituées de la formule II

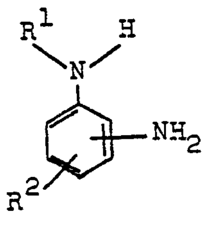

(II)

dans laquelle $R^1$ et $R^2$ possèdent la signification définie ci-dessus, dans un premier stade, à une température de − 30 °C à + 200 °C, avec du phosgène en excès, puis dans un deuxième stade, sans séparation des chlorures d'isocyanato-phényl-carbamoyle formés, de la formule III

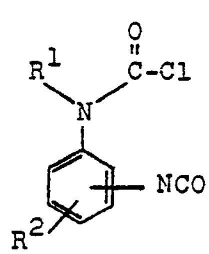

(III)

dans laquelle $R^1$ et $R^2$ possèdent la signification définie, avec des composés de la formule IV

$$H—X—R^3 \qquad (IV)$$

dans laquelle $R^3$ et X possèdent la signification définie.